# NEUE EUROPÄISCHE PATENTSCHRIFT

(11) **EP 0 971 958 B2**
(45) Veröffentlichungstag und Bekanntmachung des Hinweises auf die Entscheidung über den Einspruch: **08.02.2012**
(45) Hinweis auf die Patenterteilung: 09.04.2008
(21) Anmeldenummer: 98905132.1
(22) Anmeldetag: 27.02.1998
(51) Int. Cl.: C07K 14/755, A61K 38/37

(54) **VERFAHREN ZUR REINIGUNG VON FAKTOR VIII/VWF-KOMPLEX MITTELS KATIONENAUSTAUSCHERCHROMATOGRAPHIE**
METHOD FOR PURIFYING FACTOR VIII/VWF COMPLEX BY CATION-EXCHANGE CHROMATOGRAPHY
PROCEDE DE PURIFICATION D'UN COMPLEXE FACTEUR VIII/FACTEUR DE VON WILLEBRAND (vWF) PAR CHROMATOGRAPHIE SUR ECHANGEUR DE CATIONS

(30) Priorität: 27.02.1997 AT 33897
(43) Veröffentlichungstag der Anmeldung: 19.01.2000
(73) Patentinhaber: Baxter Innovations GmbH, 1220 Wien (AT)
(72) Erfinder: MITTERER, Artur, 2304 Orth/Donau (AT); FISCHER, Bernhard, 1160 Vienna (AT); SCHÖNBERGER, Öyvind, L., A-1180 Wien (AT); THOMAS-URBAN, Kathrin, 88709 Meersburg (DE); DORNER, Friedrich, A-1230 Wien (AT); EIBL, Johann, A-1180 Wien (AT)
(74) Vertreter: Alt, Michael
(86) Internationale Anmeldenummer: PCT/AT1998/000043
(87) Internationale Veröffentlichungsnummer: WO 1998/038220

(56) Entgegenhaltungen:
- EP-A- 0 295 645
- EP-A- 0 600 480
- EP-A2- 0 600 480
- WO-A-91/13093
- WO-A-93/15199
- WO-A-97/34930
- WO-A-97/39033
- WO-A1-97/134930
- US-A1- 4 578 218
- 'Beleg f.das allgemeineFachwissen', 01 Januar 1993, PHARMACIA Artikel 'Affinity Chromatography'
- HARRISON ET AL: 'Chromatography of the VIII/vWF Complex on Dextran Sulphate Sepharose' THROMBOSIS RESEARCH Bd. 50, 01 Januar 1988, Seiten 295 - 304
- WILLEBRAND FACTOR: 'Factor VIII', Bd. 1, 01 Januar 1989, CRC PRESS INC., FLORIDA Artikel 'Biochemical,Functional Aspects - Chapter 14: The interaction of Factor VIII/ vWF with solid matrices and ligands'

## Beschreibung

Die Erfindung betrifft ein Verfahren zur Reinigung von Faktor VIII/vWF-Komplex aus einem biologischen Ausgangsmaterial mittels Kationenaustauscherchromatographie (wobei der Kationenaustauscher nicht Heparin ist) und stufenweiser Elution, sowie gereinigten Faktor VIII/vWF-Komplex, der insbesondere hochmolekularen vWF-Multimeren, enthält.

Im Plasma zirkuliert der von Willebrand-Faktor in einer Konzentration von 5 bis 10 mg/l und zum größten Teil in Form eines nicht-kovalent gebundenen Komplexes mit Faktor VIII. Im Kryopräzipitat ist Faktor VIII/vWF-Komplex stark angereichert und kann daraus oder aus Plasma oder Plasmafraktionen mit bekannten Fraktionierungsverfahren isoliert werden.

Bei der Hämophilie ist die Blutgerinnung durch Mangel an bestimmten plasmatischen Blutgerinnungsfaktoren gestört. Bei der Hämophilie A beruht die Blutungsneigung auf einem Mangel an Faktor VIII bzw. vWF (phänotypische Hämophilie). Die Behandlung der Hämophilie A erfolgt in erster Linie durch Ersatz des fehlenden Gerinnungsfaktors durch Faktorenkonzentrate z.B. durch Infusion von Faktor VIII oder Faktor VIII/vWF-Komplex.

Für den Einsatz zur Therapie von Patienten mit Hämophilie A, aber auch von von Willebrand-Syndrom ist ein gereinigter Faktor VIII, komplexiert mit vWF, wünschenswert (Berntorp, 1994, Haemostasis 24:289-297). Insbesondere wird immer wieder betont, daß in Präparaten ohne oder nur einem geringen Gehalt an vWF, eine verlängerte Blutungszeit und eine geringe Faktor VIII:C-Halbwertszeit in vivo zu beobachten ist. Eine Normalisierung von vWF in vivo ist wichtig, um eine Konzentration von Faktor VIII im Plasma sowohl durch Reduktion der Faktor VIII-Eliminierungsrate als auch durch Unterstützung der Freisetzung von endogenem Faktor VIII, aufrecht zu erhalten (Lethagen et al., 1992, Ann. Hematol. 65: 253-259).

Die DE 3 504 385 beschreibt die Durchführung einer Ionenaustauscherchromatographie zur Reinigung von Faktor VIII/vWF-Komplex, wobei der Faktor VIII-Komplex über Sulfatgruppen gebunden und mit Citratpuffer, Calciumchlorid und NaCl-Gradient eluiert wird. Der Faktor VIII/vWF-Komplex wird dabei mit einer Konzentration von 0,5 M NaCl vom Träger eluiert.

Die EP 0 416 983 beschreibt die Gewinnung des Faktor VIII/vWF-Komplexes aus menschlichem Plasma durch eine Kombination aus Bariumchlorid- oder Aluminiumhydroxid-Fällung und Anionen-Austauscherchromatographie an DEAE-Fractogel.

Harrison et al. (Thrombosis Res., 1988; 50, 295-304) beschreibt die Reinigung von Faktor VIII/vWF-Komplex durch Chromatographie an Dextran-Sulfat-Sepharose.

Die EP 0 600 480 beschreibt ein Aufreinigungsverfahren für Faktor VIII/vWF-Komplex aus Vollplasma mittels kombinierter Anionenaustauscher/Kationenaustauscher-Chromatographie. Die Elution des an den Kationenaustauscher adsorbierten FVIII/vWF-Komplexes erfolgt dabei unter Verwendung eines Ca-haltigen Puffers mit 0,3 M NaCl in einem pH-Bereich zwischen 6,6 bis 7,0.

Die WO 96/10584 beschreibt ein Verfahren zur Gewinnung von hochreinem rekombinantem vWF mittels kombinierter Anionenaustauscher/Heparin-Affinitätschromatographie und die EP 0 705 846 die Trennung zwischen hoch- und niedermolekularen Fraktionen von rekombinantem vWF mittels Heparin-Affinitätschromatographie.

Die im Stand der Technik beschriebenen Faktor VIII-Präparate enthalten zwar zum größten Teil das gesamte vWF-Multimerpattern, jedoch variieren sie im Anteil an hochmolekularen vWF (HMW-vWF) und niedermolekularen vWF (LMW-vWF) und weisen auch sogenannte Triplett-Strukturen auf, die auf einen proteolytischen Abbau, insbesondere von HMW-vWF, hinweisen. Die Stabilität dieser Präparate ist dadurch oft begrenzt.

Es wird immer wieder betont, daß Faktor VIII/vWF-Präparationen, enthaltend im wesentlichen HMW-vWF, möglicherweise einen guten Einfluß auf die Blutungszeit hätten, da sie die primäre Funktion des vWF, die Plättchenagglutination, ausführen und eine höhere Affinität zu den Plättchenrezeptoren Glykoprotein IB und IIb/IIIa haben als niedermolekulare vWF-Multimere.

Es besteht ein Bedarf an einem Faktor VIII-Komplex mit einer ausreichenden spezifischen Aktivität an Faktor VIII:C- und vWF-Aktivität. Ein Problem bei der Gewinnung eines solchen Komplexes ist insbesondere die Abtrennung von Molekülen enthaltend niedermolekulare vWF-Multimere und Anreicherung von Komplexen mit hoher spezifischer vWF-Aktivität.

Ziel der vorliegenden Erfindung ist es daher, einen Faktor VIII/vWF-Komplex mit einer verbesserten spezifischen Aktivität und Stabilität zur Verfügung zu stellen.

Ein weiteres Ziel ist es, ein Verfahren zur Gewinnung eines solchen Faktor VIII/vWF-Komplex zur Verfügung zu stellen. Das Verfahren sollte sowohl für die Reinigung von rekombinantem als auch plasmatischem Faktor VIII/vWF-Komplex einsetzbar sein.

Erfindungsgemäß wird die Aufgabe dadurch gelöst, daß ein Verfahren zur Gewinnung von Faktor VIII/vWF-Komplex zur Verfügung gestellt wird, bei dem Faktor VIII/vWF-Komplex aus einer Proteinlösung an einen Kationenaustauscher gebunden wird - wobei der Kationenaustauscher nicht Heparin ist - und durch stufenweise, fraktionierte Elution Faktor VIII/vWF-Komplex mit verbesserter spezifischer vWF-Aktivität gewonnen wird. Die Gewinnung und Anreicherung von Faktor VIII/vWF mit verbesserter Aktivität und Stabilität erfolgt insbesondere dadurch, daß Faktor VIII/vWF-Komplex bei einer niedrigen Salzkonzentration gebunden wird, durch stufenweise Erhöhung der Salzkonzentration Fraktionen enthaltend Faktor VIII/vwF-Komplex mit niedermolekularen vWF-Multimeren, inaktiven vWF-Abbauprodukten und unspezifische Begleitproteine bei einer mittleren Salzkonzentration abgetrennt und Fraktionen enthaltend Faktor VIII/vWF-Komplex, der insbesondere hochmolekulare vWF-Multimere enthält, bei einer höheren Salzkonzentration gewonnen werden.

Faktor VIII/vWF-Komplex wird üblicherweise aufgrund seines sauren isoelektrischen Punktes (IEP = 5,5 bis 6) und seiner daraus resultierenden negativen Netto-Ladung im schwach sauren bis basischen Milieu über positiv geladene Anionenaustauscher aufgereinigt. Es war daher aufgrund der bisher beschriebenen Verfahren zur Reinigung von Faktor VIII/vWF-Komplex mittels positiv geladener Anionenaustauscher nicht zu erwarten, daß Faktor VIII/vWF-Komplex ebenfalls bei einem pH-Wert, der oberhalb des IEP des Komplexes liegt, und niedriger Salzkonzentration an eine negativ geladene Gelmatrix eines Kationenaustauschers bindet und von dieser durch Erhöhung der Salzkonzentration selektiv eluierbar ist. Es war ebenfalls nicht zu erwarten, daß durch stufenweise Elution bei einer Salzkonzentration von zwischen ≥ 250 mM und ≤ 300 mM unspezifische Begleitproteine, inaktive vWF-Abbauprodukte, Komplexkomponenten mit geringer spezifischer Aktivität, Faktor VIII/VWF-Komplex enthaltend niedermolekulare vWF-Multimere, nicht-komplexierter oder nur schwach gebundener Faktor VIII und freier Faktor VIII eluiert werden und bei einer Salzkonzentration von ≥ 350 mM insbesondere Faktor VIII/vWF-Komplex mit hochmolekularen vWF-Multimere erhalten werden.

Im Rahmen der vorliegenden Erfindung wurde festgestellt, daß mit dem erfindungsgemäßen Verfahren, ausgehend von einem unreinen biologischen Material, gereinigte Fraktionen erhalten werden, die im wesentlichen frei von kontaminierenden Nukleinsäuren sind. Dadurch werden durch das Verfahren auch Nukleinsäuren aus Proteinpräparationen entfernt. Dieser Effekt kann mit herkömmlichen Verfahren mittels Anionenaustauscher nicht gezeigt werden, da Nukleinsäuren aufgrund ihrer negativen Ladung an den Anionenaustauscher binden, sich durch Erhöhung der Salzkonzentration wieder vom Anionenaustauscher ablösen und ins Eluat gelangen.

Bei der Reinigung des Faktor VIII/vWF-Komplexes ist insbesondere zu beachten, daß, bedingt durch die Größe des vWF von 500 000 bis mehrere Millionen, nur solche Trägermaterialien gute Reinigungen und Ausbeuten liefern, die den Faktor VIII/vWF-Komplex in der Diffusion und Verteilung in den verwendeten Trägermaterialien nicht behindern. Bei der Durchführung des erfindungsgemäßen Verfahren zur Reinigung von Faktor VIII/vWF-Komplex mit hoher spezifischer Aktivität mittels Kationenaustauscher wird nicht nur eine Gelmatrix verwendet, die eine hohe Beladungskapazität besitzt, robust in der Handhabung ist und ein scharfes Elutionsprofil zeigt, sondern die sich auch im industriellen Maßstab ökonomisch einsetzen läßt. Damit wird das erfindungsgemäße Verfahren insbesondere für die Gewinnung von gereinigtem Faktor VIII/vWF-Komplex im großtechnischen Ansatz interessant.

Zur Durchführung des Verfahrens kann jeder bekannte Kationenaustauscher - mit Ausnahme von Heparin - eingesetzt werden, wobei Kationenaustauscher mit einem Sulfopropyl- oder Carboxymethyl-Gruppen konjugierten Träger bevorzugt sind. Als gut geeignet haben sich zum Beispiel SP-Sepharose^{®} Fast Flow und CM-Sepharose^{®} Fast Flow (Pharmacia), Fractogel^{®} EMD-S03 und Fractogel^{®} EMD COOH (Merck), Poros^{®} 10 SP und Poros^{®} 10 S (Perseptive Biosystems) und Toyopearl™ SP 550 C und Toyopearl^{™} CM-650 (M) (TosoHaas) erwiesen.

Als besonders günstig für die Gewinnung von gereinigtem vWF hat sich ein großporiges Gel mit Tentakelstruktur vom Typ Fractogel^{®} EMD-SO3 und Fractogel^{®} EMD COOH (Merck) erwiesen.

Die Adsorption des Faktor VIII/vWF-Komplexes an den Kationenaustauscher erfolgt vorzugsweise bei einer Salzkonzentration im Puffer von ≤ 250 mM. Bevorzugte Adsorptionspuffer weisen daher eine Salzkonzentration von 50 bis 250 mM, insbesondere im Bereich von 150 bis 250 mM (z.B. 150 mM) auf. Durch stufenweise Erhöhung der Salzkonzentration im Puffer kann selektiv Faktor VIII/vWF-Komplex enthaltend insbesondere hochmolekulare vWF-Multimere bei einer Salzkonzentration von ≥ 350 mM eluiert werden. Faktor VIII/vWF-Komplex enthaltend niedermolekulare vWF-Multimere und proteolytische vWF-Abbauprodukte, die in der Proteinlösung enthalten sind und die eine geringe spezifische Aktivität in bezug auf die vWF-Aktivität, insbesondere auf die Ristocetin-Kofaktor-Aktivität, die Kollagenbindungsaktivität und die spezifische Plättchenagglutinationsaktivität aufweisen, sowie freier Faktor VIII:C werden bei einer Salzkonzentration zwischen ≥ 250 mM und ≤ 300 mM, vorzugsweise bei 300 mM vom Kationenaustauscher eluiert und gegebenenfalls gewonnen. Diese Fraktion kann zur weiteren Reinigung beispielsweise von Faktor VIII:C, der insbesondere frei ist von plättchenagglutinierender vWF-Aktivität, eingesetzt werden.

Die Adsorption und Desorption des Faktor VIII/vWF kann in einem Puffer, enthaltend als Salz ein ein- oder zweiwertiges Metallion, erfolgen, wobei als Salz vorzugsweise NaCl verwendet wird.

Im erfindungsgemäßen Verfahren wird als Puffersystem zur Elution der an den Kationenaustauscher gebundenen Proteine eine Pufferlösung bestehend aus Puffersubstanzen, insbesondere Glycin, Phosphatpuffer oder Citratpuffer, und Salz verwendet.

Der Elutionspuffer kann einen pH-Wert im Bereich zwischen 4,5 bis 8,5, vorzugsweise zwischen ≥ 7,1 und ≤ 8,5 aufweisen.

Das erfindungsgemäße Verfahren kann als Batch-Verfahren oder als Säulenchromatographie durchgeführt werden.

Die optimalen Parameter wie Salzkonzentration, pH-Wert und Temperatur zur Durchführung des erfindungsgemäßen Verfahrens sind jedoch jeweils abhängig vom verwendeten Kationenaustauschermaterial. Es liegt jedoch im allgemeinen Wissen eines Fachmannes, die im Rahmen der vorliegenden Erfindung offenbarten Bedingungen zur Durchführung des Verfahrens für den jeweilig eingesetzen Kationenaustauschertyp zu optimieren.

Durch das erfindungsgemäße Verfahren wird insbesondere ein Faktor VIII/vWF-Komplex gewonnen und angereichert, der insbesondere hochmolekulare vWF-Multimere enthält.

Die gewonnene Faktor VIII/vWF-Komplex-Fraktion ist im wesentlichen frei von niedermolekularen vWF-Multimeren, vWF-Fragmenten mit geringer spezifischer Aktivität und kontaminierenden Nukleinsäuren.

Als Ausgangsmaterial zur Gewinnung von gereinigtem Faktor VIII/vWF-Komplex mit dem erfindungsgemäßen Verfahren kann jede Faktor VIII-Komplex-haltige Lösung eingesetzt werden. Ausgangsmaterialien sind insbesondere biologische Materialien, wie Plasma, eine Plasmafraktion, Kryopräzipitat, oder ein Überstand oder ein Extrakt einer rekombinanten Zellkultur.

Faktor VIII/vWF-Komplex-haltige Lösungen können jedoch auch angereicherte Proteinlösungen sein, die durch einen vorangegangenen Schritt, etwa über Gelfiltration, Anionenaustauscherchromatographie, Affinitätschromatographie oder einer Kombination davon, vorgereinigt oder angereichert sind.

Gemäß einer besonderen Ausführungsform des erfindungsgemäßen Verfahrens wird als Ausgangslösung eine über einen Anionenaustauscher angereicherte Faktor VIII/vWF-Komplex-haltige Fraktion eingesetzt. Bei der anschließenden Kationenaustauscherchromatographie erfolgt dann eine Reinigung und Trennung von Faktor VIII/vWF-Komplex enthaltend hochmolekulare bzw. niedermolekulare vWF-Multimere. Es sind jedoch auch andere Kombinationen, wie etwa Affinitäts-/Kationenaustauscher-Chromatographie, Anionenaustauscher-/Affinitäts-/Kationenaustauscherchromatographie möglich, um eine Anreicherung und selektive Gewinnung von Faktor VIII/vWF mit verbesserter spezifischer Aktivität und Stabilität zu erreichen.

Mit dem oben beschriebenen erfindungsgemäßen Verfahren wird Faktor VIII/vWF mit verbesserter spezifischer Aktivität aus einem unreinen Faktor VIII/vWF-haltigen Material vielfach angereichert.

Da prinzipiell jedes biologische Material mit infektiösen Keimen kontaminiert sein kann, wird zur Herstellung eines virussicheren Präparates die gewonnene Faktor VIII/vWF-haltige Fraktion zur Inaktivierung bzw. Abreicherung von Viren behandelt. Dazu können alle aus dem Stand der Technik bekannten Verfahren, wie chemisch/physikalische Methoden, Inaktivierung durch Kombination einer photoaktiven Substanz und Licht oder Abreicherung durch Filtration eingesetzt werden. Zur Inaktivierung von Viren eignet sich insbesondere eine Hitzebehandlung in Lösung bzw. in festem Zustand, welche sowohl lipidumhüllte als auch nicht-lipidumhüllte Viren verlässlich inaktivieren kann. Die Virusabreicherung erfolgt vorzugsweise durch eine Filtration über Nanofilter.

Gemäß einem weiteren Aspekt stellt die vorliegende Erfindung gereinigten Faktor VIII/vWF-Komplex, der insbesondere hochmolekulare vWF-Multimere enthält, erhältlich aus einer Faktor VIII/vWF-haltigen Lösung durch die erfindungsgemäße Kationenaustauscherchromatographie zur Verfügung. Faktor VIII/vWF mit erhöhter spezifischer vWF-Aktivität von mindestens 66 U/mg Protein und erhöhter spezifischer Faktor VIII-Aktivität von mindestens 500 U/mg Protein wird ausgehend von einem Ausgangsmaterial enthaltend u.a. Faktor VIII/vWF mit geringer Reinheit und niedriger spezifischer Aktivität angereichert und Begleitproteine, insbesondere nicht oder schwach gebundener und damit freier Faktor VIII bzw. Faktor VIII-Komplex mit geringer vWF-Aktivität, werden selektiv abgetrennt. Dadurch wird ein Faktor VIII/vWF-Komplex gewonnen, der hochmolekulare vWF-Multimere enthält und im wesentlichen frei ist von Faktor VIII-Komplex mit niedermolekularen vWF-Multimeren, vWF-Abbauprodukten, nicht-komplexiertem Faktor VIII und möglicherweise Faktor VIIIa. Der erfindungsgemäße Faktor VIII/vWF-Komplex weist aufgrund der selektiven Anreicherung von hochmolekularen vWF-Multimeren eine verbesserte Plättchenagglutinationsaktivität und eine erhöhte Stabilität auf.

Gemäß einem weiteren Aspekt wird eine gereinigte Präparation enthaltend Faktor VIII/vWF-Komplex, der insbesondere hochmolekulare vWF-Multimere enthält, oder Faktor VIII:C, im wesentlichen frei von plättchenagglutinierender vWF-Aktivität, zur Verfügung gestellt, der durch das erfindungsgemäße Verfahren erhältlich ist.

Bei Gewinnung und Herstellung der erfindungsgemäßen Präparation mit einem Ausgangsmaterial plasmatischen oder rekombinanten Ursprungs wird, wie oben beschrieben, zur Entfernung von infektiösen Partikeln gegebenenfalls ein Virusabreicherungs/oder Inaktivierungsverfahren durchgeführt, wobei eine Virusinaktivierung und/oder Virusabreicherung prinzipiell vor oder nach jedem Reinigungsschritt ausgehend vom Ausgangsmaterial bis zur hergestellten pharmazeutischen Zubereitung erfolgen kann. Damit ist die erfindungsgemäße Präparation in jedem Fall virussicher und frei von infektiösem Material.

Ein weiteres Kriterium für die Reinheit und geringe Infektiösität eines Produktes ist auch die Abwesenheit von kontaminierenden Nukleinsäuren. Die erfindungsgemäße Präparation ist daher im wesentlichen frei von Nukleinsäuren. "Im wesentlichen" bedeutet hier, daß der Gehalt an Nukleinsäure ≤ 0,7 bezogen auf das Verhältnis 260/280 nm ist. Die Nukleinsäure kann jedoch auch gemäß einem Verfahren, wie es beispielsweise in der EP 0 714 987 und der EP 0 714 988 beschrieben wird, quantifiziert werden.

Gemäß einem weiteren Aspekt liegt die erfindungsgemäße Präparation in einer lagerstabilen Form vor. Die Präparation enthaltend gereinigten Faktor VIII/vWF mit verbesserter spezifischer vWF-Aktivität kann als fertige Lösung, Lyophilisat oder tiefgefroren bereitgestellt werden. Aufgrund ihrer Reinheit ist die Präparation besonders stabil. Es hat sich gezeigt, daß die erfindungsgemäße Präparation bei -20°C für mindestens 6 Monate, bei 4°C in Lösung für mindestens 4 Wochen und als Lyophilisat für mindestens 1 Jahr stabil ist. Es zeigte sich, daß innerhalb des jeweiligen Zeitraumes die Faktor VIII/vWF-Aktivität um maximal 10% reduziert wird und das Multimermuster der vWF-Multimere keine wesentliche Änderung zeigt.

Die Formulierung der erfindungsgemäßen Präparation kann in an sich bekannter und üblicher Weise erfolgen. Der gereinigte Faktor VIII/vWF, enthalten in der erfindungsgemäßen Präparation, wird mit einem Puffer enthaltend Salze, wie NaCl, Trinatriumcitratdihydrat und/oder CaCl₂, und Aminosäuren, wie Glycin und Lysin, bei einem pH im Bereich von 6 bis 8 gemischt und als pharmazeutisches Präparat formuliert.

Die Präparation kann zur Herstellung eines Arzneimittels zur Behandlung von Patienten mit Hämophilie, phänotypischer Hämophilie und vWD verwendet werden.

Gemäß einem weiteren Aspekt betrifft die vorliegende Erfindung ein Verfahren zur Herstellung einer Faktor VIII/vWF-Komplex-Präparation aus Plasma oder einer Plasmafunktion, welche dadurch gekennzeichnet ist, daß Plasma oder eine Plasmafraktion mit einem Kationenaustauscher kontaktiert wird, wobei der Faktor VIII/vWF-Komplex adsorbiert wird, der mit Faktor VIII/vWF-Komplex beladene Kationenaustauscher gegebenenfalls gewaschen wird, anschließend der Faktor VIII/vWF-Komplex eluiert wird, wobei ein Eluat mit einer bezüglich dem Faktor VIII/vWF-Komplex mindestens 300-fachen Reinheit und einer Ausbeute an Faktor VIII/vWF-Komplex von mindestens 50% gegenüber Plasma erhalten wird, und schließlich das erhaltene Eluat zu einer Faktor VIII/vWF-Komplex-Präparation aufgearbeitet wird.

Überraschenderweise wurde im Rahmen der vorliegenden Erfindung festgestellt, daß der Faktor VIII/vWF-Komplex mit einer Kationenaustauscherchromatographie ausgehend von Plasma oder einer Plasmafraktion in hoher Reinheit und gleichzeitig mit hoher Ausbeute zur Verfügung gestellt werden kann.

Als Plasmafraktion wird beispielsweise ein Kryopräzipitat, eventuell nach vorheriger Adsorptionsbehandlung zur Entfernung von Prothrombin-Komplex, oder eine Cohn-Fraktion eingesetzt.

Das Verfahren eignet sich hervorragend zur Reinigung des Faktor VIII-Komplex aus einer Plasmafraktion im industriellen Maßstab, da aufgrund der effektiven Reinigung eine Vielzahl von weiteren Reinigungsschritten, z.B. weiteren chromatographischen Reinigungsschritten, nicht erforderlich ist. Es hat sich überraschenderweise gezeigt, daß der Faktor VIII-Komplex durch die einfache Kationenaustauscherchromatographie mit einer mindestens 300-fachen Reinheit gegenüber Plasma, vorzugsweise mindestens 400-fachen Reinheit, erhalten werden kann, mit einer gleichzeitig hohen Ausbeute von mindestens 50%, vorzugsweise mindestens 60%, bezogen auf Plasma. Es ist daher bevorzugt, das Reinigungsverfahren derart zu gestalten, daß nur eine einzige chromatographische Reinigung vorgenommen wird, nämlich die an dem Kationenaustauscher. Diese chromatographische Reinigung wird üblicherweise als terminaler Reinigungsschritt vorgenommen, bevor der Faktor VIII-Komplex zu einer pharmazeutischen Präparation formuliert wird.

Üblicherweise wird das Ausgangsmaterial in einem kalziumhältigen Puffer auf den Kationenaustauscher aufgetragen. Unmittelbar vor dem Auftragen bietet sich auch eine Maßnahme zur Inaktivierung von potentiell vorhandenen Viren an, wie humanpathogene Viren, die durch Blut übertragbar sind. Dabei ist eine Behandlung mit einem viriziden Detergens bzw. einem organischen Lösungsmittel und/oder Detergens bevorzugt. Eine Behandlung mit Triton oder Tween in Gegenwart von TNBP (Tri(n-butyl)phosphat) wird beispielsweise gemäß der EP 0 131 740 durchgeführt. Durch die anschließende Kationenaustauscherchromatographie wird das viruzide Mittel effektiv entfernt. Falls der adsorbierte Komplex gewaschen wird, wird diese Waschung bevorzugterweise mit einem Waschpuffer durchgeführt, dessen Ionenstärke über der des Adsorptionspuffers liegt, beispielsweise 10 bis 30% höher. Zur Elution des Faktor VIII/vWF-Komplexes wird vorzugsweise die Ionenstärke weiter erhöht. Die Elution des Faktor VIII/vWF-Komplexes wird durch die Erhöhung der Ionenstärke erreicht, welche vorzugsweise um mindestens 50%, am meisten bevorzugt um mindestens 100%, gegenüber der Ionenstärke der Ausgangslösung erhöht ist. Der Elutionspuffer enthält vorzugsweise Natriumchlorid. Zur Formulierung einer pharmazeutischen Faktor VIII/vWF-Komplex-Präparation wird üblicherweise diafiltriert und sterilfiltriert, sowie gegebenenfalls lyophilisiert.

Die Aktivität des Faktor VIII bzw. vWF wird durch die Kationenaustauscherchromatographie kaum beeinträchtigt. Es hat sich herausgestellt, daß die Ausbeute des Faktor VIII-Komplexes von mehr als 90% bezogen auf die Aktivität vor der Chromatographie gewährleistet ist. Deshalb kann auch während der chromatographischen Reinigung auf übliche Stabilisatoren des Faktor VIII, wie z.B. Antithrombin III und/oder Heparin verzichtet werden.

Im Gegensatz zu im Stand der Technik bekannten Verfahren, in welchen die Kationenaustauscherchromatographie stets nur für bereits aufgereinigte Faktor VIII/vWF-Komplex-Präparationen in Erwägung gezogen worden ist (vgl. EP 0 600 480-A2), hat sich erfindungsgemäß gezeigt, daß sich die Kationenaustauscherchromatographie ausgezeichnet zur direkten Reinigung von Faktor VIII/vWF-Komplex aus Plasma oder einer (rohen) Plasmafraktion eignet. Mit einem derartigen Verfahren ist es nicht einmal notwendig, zur Herstellung einer Faktor VIII/vWF-Komplex-Präparation weitere chromatographische Verfahren vorzusehen, da die Reinheit, die durch die Kationenaustauscherchromatographie ausgehend von Plasma oder einer Plasmafraktion erzielt wird, bereits den Anforderungen an kommerzielle Faktor VIII/vWF-Komplex-Präparate genügt.

Die Erfindung wird anhand der nachfolgenden Beispiele und der Zeichnungsfiguren näher erläutert, wobei sie jedoch nicht auf diese Ausführungsbeispiele beschränkt ist.

Es zeigen:
- Figur 1:: vWF-Multimer-Analyse von Faktor VIII/vWF-Komplex aus Kryopräzipitat vor und nach Reinigung mit Kationenaustauscher
- Figur 2:: vWF-Multimer-Analyse von Faktor VIII/vWF-Komplex aus Kryopräzipitat vor und nach Reinigung mittels kombinierter Anionen/Kationenaustauscherchromatographie

Beispiel 1 beschreibt die Reinigung von plasmatischem Faktor VIII/vWF-Komplex durch Kationenaustauscherchromatographie und stufenweiser Elution; Beispiel 2 beschreibt die Reinigung von Faktor VIII/vWF-Komplex durch Kombination von Anionen-/Kationenaustauscherchromatographie und stufenweiser Elution vom Kationenaustauscher; Beispiel 3 beschreibt die Reinigung von rvWF/rFaktor VIII-Komplex mittels Kationenaustauscher; Beispiel 4 beschreibt die Isolierung des Faktor VIII/vWF-Komplexes über Kationenaustausch.

### Beispiel 1:

### Reinigung von plasmatischem FVIII-Komplex durch KationenAustauscherchromatographie

Kryopräzipitat aus humanem Plasma wurde in Natrium-Acetat-Puffer, pH 7, aufgelöst und es wurden 20 Einheiten Heparin pro ml Lösung zugegeben. Pro 1 g Kryopräzipitat wurden 0,25 ml 2% Al(OH)₃-Suspension zugegeben, und für 30 Minuten inkubiert. Anschließend wurde für 20 Minuten bei 10000 RPM zentrifugiert, um ein trübungsfreies Kryopräzipitat zu erhalten.

Eine Chromatographiesäule wurde mit Fractogel® EMD-S03 gefüllt und mit Puffer (30 mM Glycin-NaCl-Puffer) gespült. Anschließend wurde aufgelöstes Kryopräzipitat durch die Kationenaustauschersäule filtriert und im Durchfluß wurden solche Proteine erhalten, die nicht an den Austauscher binden (Fraktion 1). Unspezifisch gebundene Proteine wurden durch Spülen der Säule mit 0,3 M NaCl in Puffer entfernt (Fraktion 2). Anschließend wurde FVIII/vWF-Komplex von der Austauschersäule durch Elution mit 0,4 M bzw. 0,5 M NaCl eluiert (Fraktion 3 bzw. Fraktion 4).

Aus der Tabelle 1 ist ersichtlich, daß sowohl vWF als auch FVIII durch den Kationenaustauscher gebunden wurden. Durch Spülen der Kationenaustauschersäule mit 0,3 M NaCl (Fraktion 2) wurden kein vWF und nur 10 % der FVIII-Aktivität erhalten. Durch diese Elutionsstufe wurde der FVIII, der nicht als Komplex mit funktionell aktivem vWF vorlag, abgetrennt. Durch anschließende Desorption mit 0,4 M NaCl (Fraktion 3) wurde FVIII/vWF-Komplex erhalten, der etwa 20 % des funktionell aktiven vWF und etwa 30 % der Gesamtmenge an FVIII enthielt. Der restliche FVIII-Komplex wurde anschließend mit 500 mM NaCl vom Kationenaustauscher eluiert (Fraktion 4). Fraktion 4 enthielt Faktor VIII/vWF-Komplex, der 80 % der vWF-Aktivität und 50 % der FVIII-Aktivität ausgehend vom Kryopräzipitat beinhaltet. Durch die Kationenaustauscherchromatographie kam es zu einer 20-fachen Reinigung von FVIII (spezifische Aktivität: 12 IU FVIII:C/mg Protein) gegenüber Kryopräzipitat bzw. zu einer 350-fachen Aufreinigung von FVIII bezogen auf Plasma (Fraktion 4). Aus der Fraktion 3 kann FVIII gewonnen werden.

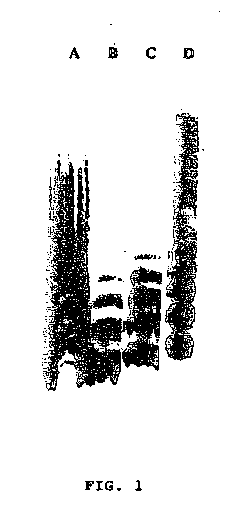

Figur 1 zeigt die vWF-Multimer-Analyse von Faktor VIII/vWF-Komplex vor und nach Reinigung mit Kationenaustauscher, wobei Spur A das vWF-Multimermuster des Kryopräzipitats, Spur B des 300 mM NaCl-Eluats (Fraktion 2, Tabelle 1), Spur C des 400 mM NaCl-Eluats (Fraktion 3, Tabelle 1) und Spur D des 500 mM Eluats (Fraktion 4, Tabelle 1) darstellt. Aus Figur 1 ist ersichtlich, daß durch die Kationenaustauscherchromatographie ein Faktor VIII/vWF-Komplex mit hochmolekularen vWF-Multimerstruktur erhalten wird. Faktor VIII/vWF-Komplex enthaltend niedermolekulare vWF-Multimere wird entweder nicht an den Kationenaustauscher gebunden (Fraktion 1) oder bei der Elution mit 0,3 M NaCl abgetrennt (Fraktion 2).

**Tabelle 1: Reinigung von FVIII/vWF-Komplex aus Kryopräzipitat mittels Kationenaustauscherchromatographie**

| **Probe** | **vWF:Risto-CoF-Aktivität (U/ml)** | **FVIII:C-Aktivität (U/ml)** |
|---|---|---|
| Kryopräzipitat | 2,2 | 2,4 |
| Fraktion 1 (Nicht gebunden) | 0 | 0 |
| Fraktion 2 (Eluat 300 mM NaCl) | 0 | 0,1 |
| Fraktion 3 (Eluat 400 mM NaCl) | 1,8 | 3,6 |
| Fraktion 4 (Eluat 500 mM NaCl) | 1,8 | 1,5 |

### Beispiel 2:

### Reinigung von plasmatischem FVIII/vWF-Komplex durch Kombination von Anionen-/Kationenaustauscherchromatographie

Kryopräzipitat aus humanem Plasma wurde in einem Puffer aus 7 mM Tris, 100 mM Na-Acetat, 100 mM Lysin, 120 mM NaCl bei pH 6,7 aufgelöst. Zur Vorbehandlung wurde Al(OH)₃ eingerührt. Anschließend wurde der Niederschlag durch Zentrifugation abgetrennt.

Auf diese Weise vorbehandeltes Kryopräzipitat wurde auf eine Säule Fractogel® EMD-TMAE aufgetragen. Nicht gebundene Proteine wurde durch Spülen der Säule mit Lösungspuffer erhalten (Fraktion 1). Diese Fraktion 1 enthielt 60 % der vWF-Aktivität, jedoch nur 10 % der FVIII-Aktivität. Durch Elution der Säule mit 400 mM NaCl (Fraktion 2) wurde anschließend FVIII/vWF-Komplex erhalten. Fraktion 2 enthielt die restliche vWF-Aktivität und 70 % der FVIII-Aktivität ausgehend vom Kryopräzipitat.

**Tabelle 2: Reinigung von FVIII-vWF-Komplex durch Kombination von Anionen- und Kationenaustauscherchromatographie**

| **probe** | **vWF:RistoCoF-Aktivität (U/ml)** | **FVIII:C Aktivität (U/ml)** |
|---|---|---|
| Kryopräzipitat | 12,5 | 12,2 |
| Fraktion 1 (nicht gebunden) | 3,5 | 0,7 |
| Fraktion 2 (Eluat 400 mM NaCl) | 2,5 | 14,5 |

Der FVIII/vWF-Komplex der Fraktion 2 wurde mit 20 mM Glycin/NaCl-Puffer 4-fach verdünnt, und anschließend auf eine Kationenaustauschersäule Fractogel^{®} EMD-SO3 aufgetragen. Nicht-bindende Proteine wurden in der Fraktion 1 erhalten. Schwach gebundene Proteine wurden durch Spülen der Säule mit 200 mM NaCl entfernt (Fraktion 2). Anschließend wurde stufenweise mit 400 mM NaCl (Fraktion 3) und 500 mM NaCl (Fraktion 4) eluiert. In den Fraktionen 3 und 4 wurden jeweils 45 % der vWF-Aktivität und 55 % bzw. 40 % der FVIII-Aktivitäten gefunden.

**Tabelle 3: Reinigung von FVIII/vWF-Komplex durch Kombination von Anionen- und Kationenaustauscherchromatographie**

| **Probe** | **vWF:RistoCoF Aktivität (U/ml)** | **FVIII:C Aktivität (u/ml)** |
|---|---|---|
| Ausgang | 0,63 | 3,6 |
| Fraktion 1 (nicht gebunden) | 0 | 0 |
| Fraktion 2 (Eluat 200 mM NaCl) | 0 | 0 |
| Fraktion 3 (Eluat 400 mM NaCl) | 0,3 | 2,26 |
| Fraktion 4 (Eluat 500 mM NaCl) | 0,25 | 1,43 |

Aus der Tabelle 3 ist ersichtlich, daß sowohl vWF als auch FVIII durch den Kationenaustauscher gebunden werden. Durch Spülen der Kationenaustauschersäule mit 0,2 M NaCl (Fraktion 2) wurde kein aktiver vWF und kein FVIII gefunden. Der FVIII/vWF-Komplex wurde anschließend in den Fraktionen 3 und 4 eluiert.

Während die spezifische Aktivität des FVIII:C im Kryopräzipitat 0,59 U/mg Protein betrug, beträgt die spezifische Aktivität des FVIII:C in den Fraktionen 3 und 4 500 U/mg Protein bzw. 477 U/mg Protein. Die spezifische Aktivität des vWF stieg ausgehend von Kryopräzipitat von 0,6 U/mg Protein auf 66 U/mg Protein und 83 U/mg Protein in den Fraktionen 3 und 4.

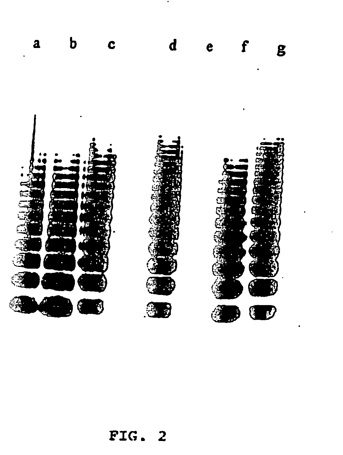

Figur 2 zeigt die vWF-Multimer-Analyse von Faktor VIII/vWF-Komplex vor und nach Reinigung mit kombinierter Anionen-/Kationenaustauscherchromatographie, wobei die Spuren a bis c die Chromatographie am Anionenaustauscher und die Spuren d bis g am Kationenaustauscher zeigen. Fig. 2 zeigt in Spur a das vWF-Multimermuster des Kryopräzipitats, Spur b des Durchlaufs (Fraktion 1, Tabelle 2), Spur c des 400 mM NaCl-Eluats (Fraktion 2, Tabelle 2), Spur d des 400 mM NaCl-Eluats (Fraktion 2, Tabelle 2) vor Kationenaustauscher, Spur e des 200 mM NaCl-Eluats (Fraktion 2, Tabelle 3), Spur f des 400 mM NaCl-Eluats (Fraktion 3, Tabelle 3) und Spur g des 500 mM NaCl-Eluats (Fraktion 4, Tabelle 3).

### Beispiel 3:

### Reinigung eines r-vWF/r-FVIII-Komplexes mittels Kationenaustauscherchromatographie (Derzeit nach Ansicht der Anmelderin der beste Weg zur Ausführung der Erfindung)

1000 ml eines Zellkulturüberstandes, enthaltend rekombinanten rFVIII/rvWF-Komplex, wurden auf eine Säule, gefüllt mit 20 ml Fractogel® TSK-S03, aufgetragen. Nach dem Waschen der Säule mit Puffer, pH 7,4, mit 250 mM NaCl, wurde der gebundene rFVIII/rvWF-Komplex mit einem Puffer, pH 7,4, mit 600 mM NaCl eluiert. In Tabelle 4 sind die Ergebnisse dieses Säulenlaufes dargestellt.

**Tabelle 4: Reinigung von rekombinanten rFVIII/rvWF-Komplex mittels Kationenaustauscherchromatographie**

| **Probe** | **FVIII:C Aktivität (U/ml)** | **vWF:RistoCoF-Aktivität (U/ml)** |
|---|---|---|
| Ausgang | 2,3 | 0,1 |
| Durchfluß | 0,1 | 0 |
| 250 mM NaCl-Eluat | 0,2 | 0 |
| 600 mM NaCl-Eluat | 85 | 4,4 |

Das Beispiel zeigt, daß ein Komplex bestehend aus rekombinantem FVIII und rekombinantem vWF (der normalerweise während der Fermentation von rekombinantem FVIII anfällt) an einen Kationenaustauscher bindet und selektiv durch Erhöhung der Salzkonzentration, von Begleitproteinen getrennt, eluiert werden kann.

In dem Beispiel wurde ein rFVIII/rvWF-Komplex mit einer spezifischen FVIII-Aktivität von 130 U/mg Protein in einer Ausbeute von 75% erhalten. Dies entspricht einem Reinigungsfaktor von 28 für diesen Schritt. Die spezifische Aktivität des r-vWF hängt stark von der Qualität des exprimierten r-vWF ab. In diesem Fall lag sie bei 7 U/mg im Eluat, was einem Reinigungsfaktor von 35 entspricht.

Durch Variation des rFVIII/rvWF-Verhältnisses im Ausgang, oder durch Anschließen eines weiteren chromatographischen Schrittes, kann die spezifische Aktivität des FVIII:C noch weiter verbessert werden.

### Beispiel 4 :

### Isolierung des FVIII/vWF-Komplexes über Kationenaustausch

### Beispiel 4A:

210 g Kryopräzipitat werden in 950 ml CaCl₂-heparinhältigem Citratpuffer gelöst und auf pH 6,0 gestellt. Unlösliche Anteile, hauptsächlich Fibrinogen,wurden abtrennt. Zur Inaktivierung von eventuell vorhandenen pathogenen Viren wurde die klare Lösung mit 1 % Triton X100 und 0,3 % TNBP (Tri(n-butyl)phosphat behandelt. 100 ml Fractogel EMD-SO₃⁻-650(M) der Fa. Merck, Darmstadt (DE), wurden zur Adsorption des virusinaktivierten FVIII herangezogen, das zuvor bei pH 6,0 in einer acetatgepufferten NaCl-Lösung mit einer Leitfähigkeit von 10 mS/cm äquilibriert wurde. Der FVIII wurde durch Erhöhung der Ionenstärke auf 500 mM NaCl vom Gel abeluiert; vorher wurde eine Waschung mit 500 ml 150 mM NaCl-Lösung durchgeführt.

### Beispiel 4B:

Statt Fractogel EMD-SO₃⁻ wurde in diesem Beispiel Toyopearl SP-550C verwendet.

### Ergebnisse :

| | Ausbeute/Plasma | | Reinheitsgrad gegenüber Plasma |
|---|---|---|---|
| | FVIIIc | FvWF | |
| Beispiel 4A | 62 % | 68 % | 450 X |
| Beispiel 4B | 56 % | 62 % | 370 X |

FVIIIc und FvWF sind in derselben Fraktion gewonnen worden.

## Patentansprüche

1. Verfahren zur Gewinnung von Faktor VIII/vWF-Komplex, **dadurch gekennzeichnet, dass** Faktor VIII/vWF-Komplex aus einer Proteinlösung an einen Kationenaustauscher gebunden und durch stufenweise Erhöhung der Salzkonzentration Faktor VIII/vWF-Komplex, der insbesondere hochmolekulare vWF-Multimere enthält, gewonnen wird, wobei der Kationenaustauscher nicht Heparin ist.

2. Verfahren nach Anspruch 1, **dadurch gekennzeichnet, dass** Faktor VIII/vWF-Komplex bei einer Salzkonzentration von 50mM bis 250mM an einen Kationenaustauscher gebunden wird und Faktor VIII/vWF-Komplex, enthaltend niedermolekulare vWF-Multimere, und Faktor VIII:C frei von plättchenagglutinierender vWF-Aktivität bei einer Salzkonzentration zwischen ≥ 250 mM und ≤ 300 mM eluiert und gewonnen wird.

3. Verfahren nach Anspruch 1 oder 2, **dadurch gekennzeichnet, dass** Faktor VIIVvWF-Komplex, enthaltend insbesondere hochmolekulare vWF-Multimere, durch stufenweise Fraktionierung bei einer Salzkonzentration von ≥ 350 mM gewonnen wird.

4. Verfahren nach Anspruch 3, **dadurch gekennzeichnet, dass** ein Faktor VIII/vWF-Komplex-haltige Fraktion gewonnen wird, die insbesondere frei ist von niedermolekularen vWF-Multimeren, vWF-Abbauprodukten, nicht-komplexierten oder schwach an vWF gebundenem Faktor VIII und kontaminierenden Nukleinsäuren.

5. Verfahren nach einem der Ansprüche 1 bis 4, **dadurch gekennzeichnet, dass** die Elution der Polypeptide vom Kationenaustauscher in einem Puffersystem mit einem pH-Wert im Bereich zwischen 4,5 und 8,5, vorzugsweise ≥ 7,1 und ≤ 8,5 erfolgt.

6. Verfahren nach einem der Ansprüche 1 bis 5, **dadurch gekennzeichnet, dass** der Kationenaustauscher ein Sulfopropyl- oder Carboxymethyl-Gruppen konjugierter Träger ist.

7. Verfahren nach einem der Ansprüche 1 bis 6, **dadurch gekennzeichnet, dass** ein Faktor VIII/vWF-Komplex enthaltend insbesondere hochmolekulare vWF-Multimere gewonnen wird.

8. Verfahren nach einem der Ansprüche 1 bis 7, **dadurch gekennzeichnet, dass** Faktor VIII/vWF-Komplex aus Plasma, einer Plasmafraktion, Kryopräzipitat, dem zellfreien Überstand oder Extrakt einer rekombinanten Zellkultur, oder einer angereicherten Proteinfraktion gewonnen wird.

9. Faktor VIII/vWF-Komplex, enthaltend insbesondere hochmolekulare vWF-Multimere, erhältlich aus einer Faktor VIII/vWF-haltigen Lösung durch Kationenaustauscherchromatographie nach einem Verfahren nach einem der Ansprüche 1 bis 8, wobei der Komplex eine spezifische vWF-Aktivität von mindestens 66 U/mg Protein und eine spezifische Faktor VIII-Aktivität von mindestens 500 U/mg Protein aufweist.

10. Faktor VIII/vWF-Komplex nach Anspruch 9, **dadurch gekennzeichnet, dass** er insbesondere frei ist von niedermolekularen vWF-Multimeren, inaktiven vWF-Abbauprodukten, nicht komplexiertem Faktor VIII und von Faktor VIIIa-Aktivität.

11. Präparation enthaltend Faktor VIII/vWF-Komplex gemäß Anspruch 9 oder 10, **dadurch gekennzeichnet, dass** sie virussicher und frei von infektiösem Material ist.

12. Präparation nach Anspruch 11, **dadurch gekennzeichnet, dass** sie in einer lagerstabilen Form vorliegt.

13. Präparation nach einem der Ansprüche 11 oder 12, **dadurch gekennzeichnet, dass** sie als pharmazeutisches Präparat formuliert ist.

14. Verwendung einer Präparation nach einem der Ansprüche 11 bis 13 zur Herstellung eines Arzneimittels zur Behandlung von Patienten mit Hämophilie A, phänotypischer Hämophilie und vWD.

## Claims

1. A method for recovering a factor-VIII/vWF complex, **characterised in that** the factor-VIII/vWF complex from a protein solution is bound to a cation exchanger, and by stepwise increase in the salt concentration, the factor-VIII/vWF complex is recovered which, in particular, contains high-molecular weight vWF multimers, the cation exchanger not being heparin.

2. A method according to claim 1, **characterised in that** the factor-VIII/vWF complex is bound to a cation exchanger at a salt concentration of from 50 mM to 250 mM, and **in that** the factor-VIII/vWF complex, which contains low-molecular weight vWF multimers, and factor VIII:C free from platelet agglutinating vWF activity are eluted and recovered at a salt concentration of between ≥ 250 mM and ≤ 300 mM.

3. A method according to either claim 1 or claim 2, **characterised in that** the factor-VIII/vWF complex which, in particular, contains high-molecular weight vWF multimers is recovered by stepwise fractionation at a salt concentration of ≥ 350 mM.

4. A method according to claim 3, **characterised in that** a fraction containing factor-VIII/vWF complex is recovered which, in particular, is free from low-molecular weight vWF multimers, vWF degradation products, non-complexed factor VIII, or factor VIII weakly bound to vWF, and from contaminating nucleic acids.

5. A method according to any one of claims 1 to 4, **characterised in that** elution of the polypeptides from the cation exchanger is effected in a buffer system with a pH value in the range of between 4.5 and 8.5, preferably ≥ 7.1 and ≤ 8.5.

6. A method according to any one of claims 1 to 5, **characterised in that** the cation exchanger is a sulfopropyl-group conjugated or carboxymethyl-group conjugated carrier.

7. A method according to any one of claims 1 to 6, **characterised in that** a factor-VIII/vWF complex is recovered which, in particular, contains high-molecular weight vWF multimers.

8. A method according to any one of claims 1 to 7, **characterised in that** a factor-VIII/vWF complex is recovered from plasma, a plasma fraction, a cryoprecipitate, the cell-free supernatant or extract of a recombinant cell culture, or an enriched protein fraction.

9. A factor-VIII/vWF complex which, in particular, contains high-molecular weight vWF multimers, which is obtainable from a solution containing factor-VIII/vWF by cation-exchanger chromatography according to a method as set forth in any one of claims 1 to 8, wherein the complex has a specific vWF activity of at least 66 U/mg of protein and a specific factor-VIII activity of at least 500 U/mg of protein.

10. A factor-VIII/vWF complex according to claim 9, **characterised in that** it is, in particular, free from low-molecular weight vWF multimers, inactive vWF-degradation products, non-complexed factor VIII and from factor-VIIIa activity.

11. A preparation containing factor-VIII/vWF complex according to claim 9 or 10, **characterised In that** it is safe from viruses and free from infectious material.

12. A preparation according to claim 11, **characterised in that** it is present in a storage-stable form.

13. A preparation according to any one of claims 11 or 12, **characterised in that** it is formulated as a pharmaceutical preparation.

14. Use of a preparation according to any one of claims 11 to 13 for producing a medicament for treatment of patients suffering from haemophilia A, phenotypic haemophilia and vWD.

## Revendications

1. Procédé pour l'obtention du complexe facteur VIII/vWF, **caractérisé en ce que** le complexe facteur VIII/vWF est, à partir d'une solution protéique, lié à un échangeur de cations et obtenu par élévation progressive de la concentration saline du complexe facteur VIII/vWF, lequel contient en particulier des multimères vWF de haut poids moléculaire, l'échangeur de cations n'étant pas l'héparine.

2. Procédé selon la revendication 1, **caractérisé en ce que** le complexe facteur VIII/vWF est fixé sur un échangeur cationique à une concentration saline de 50 mM à 250 mM, et **en ce que** le complexe facteur VIII/vWF, contenant des multimères vWF de bas poids moléculaires et le facteur VIII:C exempt de l'activité vWF d'agglutination de plaquettes sont élués et obtenus à une concentration saline comprise entre 250 mM et 300 mM.

3. Procédé selon la revendication 1 ou 2, **caractérisé en ce que** le complexe facteur VIII/vWF contenant en particulier des multimères vWF de haut poids moléculaire est obtenu par fractionnement progressif à une concentration saline ≥ 350 mM.

4. Procédé selon la revendication 3, **caractérisé en ce qu'**une fraction contenant le complexe facteur VIII/vWF est obtenue, laquelle est en particulier exempte de multimères vWF de bas poids moléculaire, de produits de décomposition vWF, d'acides nucléiques non complexés ou faiblement liés au facteur VIII/vWF et contaminants.

5. Procédé selon l'une quelconque des revendications de 1 à 4, **caractérisé en ce que** l'élution des polypeptides de l'échangeur cationique a lieu dans un système tampon à une valeur pH comprise dans la plage allant de 4,5 à 8,5, de préférence à une valeur pH ≥ 7,1 et ≤ 8,5.

6. Procédé selon l'une quelconque des revendications de 1 à 5 **caractérisé en ce que** l'échangeur de cations est un porteur conjugué de groupes sulfopropyles ou carboxyméthyles.

7. Procédé selon l'une quelconque des revendications de 1 à 6, **caractérisé en ce qu'**un complexe facteur VIII/vWF contenant en particulier des multimères vWF de haut poids moléculaires est obtenu.

8. Procédé selon l'une quelconque des revendications de 1 à 7, **caractérisé en ce que** le complexe facteur VIII/vWF est obtenu à partir de plasma, d'une fraction de plasma, d'un cryoprécipité, d'une couche supérieure exempte de cellules ou d'un extrait d'une culture cellulaire recombinante, ou d'une fraction protéique enrichie.

9. Complexe facteur VIII/vWF contenant en particulier des multimères vWF de haut poids moléculaires, pouvant être obtenu à partir d'une solution contenant un facteur VIII/vWF par chromatographie d'échange de cations selon un procédé d'après une quelconque des revendications 1 à 8, le complexe présentant une activité vWF spécifique d'au moins 66 U/mg de protéine et une activité facteur VIII spécifique d'au moins 500 U/mg de protéine.

10. Complexe facteur VIII/vWF selon la revendication 9, **caractérisé en ce qu'**il est en particulier exempt de multimères vWF de bas poids moléculaires, de produits de décomposition vWF inactifs, d'un facteur VIII non complexé et d'activité de facteur VIIIa.

11. Préparation contenant le complexe facteur VIII/vWF selon la revendication 9 ou 10, **caractérisée en ce qu'**elle est résistante aux virus et exempte de matière infectieuse.

12. Préparation selon la revendication 11, **caractérisée en ce qu'**elle se présente sous une forme stable au stockage.

13. Préparation selon l'une quelconque des revendications 11 ou 12, **caractérisée en ce qu'**elle est formulée en tant que préparation pharmaceutique.

14. Emploi d'une préparation selon l'une quelconque des revendications de 11 à 13 pour la fabrication d'un médicament pour le traitement de patients atteints d'hémophilie A, d'hémophilie phénotypique et de la maladie vWD.
